(19) Europäisches Patentamt
European Patent Office
Office européen des brevets

(11) Veröffentlichungsnummer: **0 103 800**
**A2**

# (12) EUROPÄISCHE PATENTANMELDUNG

(21) Anmeldenummer: 83108727.5

(22) Anmeldetag: 05.09.83

(51) Int. Cl.³: G 03 F 7/10
C 07 C 117/00, C 07 C 147/06
C 07 C 149/32

(30) Priorität: 16.09.82 DE 3234301

(43) Veröffentlichungstag der Anmeldung: 28.03.84 Patentblatt 84/13

(84) Benannte Vertragsstaaten: DE FR GB NL

(71) Anmelder: **Merck Patent Gesellschaft mit beschränkter Haftung**
**Frankfurter Strasse 250**
**D-6100 Darmstadt(DE)**

(72) Erfinder: **Haas, Günther, Dr.**
**Saarstrasse 109**
**D-6903 Neckargemünd(DE)**

(72) Erfinder: **Neisius, Karl Heinz, Dr.**
**Kattreinstrasse 76**
**D-6100 Darmstadt(DE)**

## (54) Neue Bisazidoverbindungen, diese enthaltende lichtempfindliche Zusammensetzungen und Verfahren zur Erzeugung von Reliefstrukturen.

(57) Neue Bisazidoverbindungen der Formel I

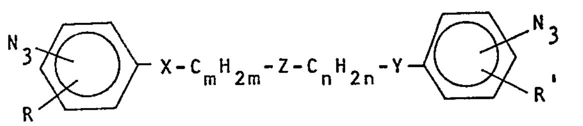

I,

worin

X und Y die gleich oder verschieden sein können, O, S, $SO_2$, $N-R_1$ oder eine Bindung,

m und n die gleich oder verschieden sein können, ganze Zahlen von 1 bis 8, mit der Maßgabe, daß die Summe von m + n nicht größer als 12 ist,

Z eine Bindung oder, falls X und Y beide eine Bindung bedeuten oder m und n beide ungleich 1 sind, auch O, S, $SO_2$ oder $N-R_1$, oder, falls X und Y beide eine Bindung bedeuten, auch CO, gegebenenfalls in 2-Stellung durch eine Oxogruppe substituiertes 1,3-Cyclopentylen oder gegebenenfalls in 2-Stellung durch eine Oxogruppe und/oder in 5-Stellung durch Alkylmit 1 bis 6 C-Atomen substituiertes 1,3-Cyclohexylen,

R und R' die gleich oder verschieden sein können, H, Halogen oder Alkyl mit 1 bis 6 C-Atomen und

$R_1$ H oder Alkyl mit 1 bis 6 C-Atomen

bedeuten,

mit der Maßgabe, daß $-X-C_mH_{2m}-Z-C_nH_{2n}-Y-$ nicht $-(CH_2)_2-$ oder $-(CH_2)_3-$ bedeutet, weisen eine hohe Empfindlichkeit für fernes UV-Licht auf und eignen sich somit als lichtempfindliche Komponenten in negativ arbeitenden, für fernes UV-Licht empfindlichen Zusammensetzungen. Die mit Hilfe der erfindungsgemäßen lichtempfindlichen Zusammensetzungen fotolithographisch hergestellten Reliefstrukturen zeichnen sich durch hervorragende mechanische Eigenschaften aus und weisen eine hohe Auflösung auf. Sie eignen sich zur fotolithographischen Herstellung von miniaturisierten Schaltungen.

EP 0 103 800 A2

Merck Patent Gesellschaft
mit beschränkter Haftung
D a r m s t a d t

# Neue Bisazidoverbindungen, diese enthaltende lichtempfindliche Zusammensetzungen und Verfahren zur Erzeugung von Reliefstrukturen

Die vorliegende Erfindung betrifft neue Bisazidoverbindungen, deren Verwendung und diese Verbindungen enthaltende negativ arbeitende, für fernes UV-Licht empfindliche Zusammensetzungen sowie ein Verfahren zur fotolithographischen Herstellung von Reliefstrukturen mit Hilfe dieser Zusammensetzungen.

Angesichts der voranschreitenden Miniaturisierung und der Packungsdichte von Festkörperbausteinen in der Halbleiterindustrie ist es erforderlich, feine Reliefstrukturen hoher Effizienz zu bilden. Zur Erzeugung derartiger feinster Reliefstrukturen, etwa zur fotolithographischen Herstellung von miniaturisierten Schaltungen oder anderen elektronischen Bauelementen, sind lichtempfindliche Zusammensetzungen erforderlich. An diese werden

hohe Anforderungen gestellt im Hinblick auf den gewünschten Auflösungsgrad der Reliefstrukturen etwa im Bereich von 0,5 bis 2 µm. Herkömmliche lichtempfindliche Zusammensetzungen sind gewöhnlich nur im Spektralbereich von 320 bis 450 nm ausreichend empfindlich. In diesem Wellenlängenbereich treten jedoch bei der Anwendung von Masken an deren Rändern Beugungs- und Interferenzerscheinungen auf, wodurch der geforderte hohe Auflösungsgrad der Reliefstrukturen nicht erreichbar ist. Zur Herstellung hochfeiner Reliefstrukturen verwendet man daher Licht im Wellenlängenbereich von 200 bis 320 nm, sogenanntes "fernes UV-Licht", das an feinen Masken weniger starke Beugungs- und Interferenzeffekte hervorruft und daher die Erzeugung feinerer Reliefstrukturen gestattet.

Demzufolge mußten die lichtempfindlichen Zusammensetzungen dem verwendbaren Wellenlängenbereich des Lichtes angepaßt werden, d.h. sie müssen im "fernen UV-Bereich" empfindlich sein.

In der DE-OS 29 48 324 werden lichtempfindliche Massen beschrieben, die im Wellenlängenbereich von 200 bis 300 nm empfindlich sind. Diese enthalten eine Bisazidoverbindung, worin die Azidogruppen jeweils an aromatische Kerne gebunden sind, welche ihrerseits nur durch eine kurze Brücke wie etwa -O-, -S-, -S-S-, $-SO_2-$, $-CH_2-$ oder $-CH_2CH_2-$ miteinander verknüpft werden. Außerdem enthalten diese lichtempfindlichen Massen polymere Verbindungen, die mit dem Produkt der fotochemischen Reaktion der Bisazidoverbindung vernetzbar sind.

Außerdem wurde die Verwendung von Poly-methylmethacrylat oder Poly-methylisoprenylketon als lichtempfindliche Zusammensetzung mit einer Empfindlichkeit im Wellenlängenbereich von 200 bis 320 nm vorgeschlagen.

Die bisher verwendeten und vorgeschlagenen lichtempfindlichen Zusammensetzungen lassen jedoch noch viel zu wünschen übrig. Insbesondere sind die Bestandteile der lichtempfindlichen Zusammensetzungen oft untereinander nicht genügend stabil und miteinander verträglich, oder die nach Belichtung und Entwicklung erhaltenen Reliefstrukturen weisen ungenügende mechanische Eigenschaften auf, was sich nachteilig z.B. auf das Auflösungsvermögen und das Kantenprofil oder die Resistenz gegen saure oder alkalische Ätzmedien auswirkt. Darüberhinaus ist die Lichtempfindlichkeit derartiger Zusammensetzungen für die Anforderungen der Praxis oft viel zu gering.

Der vorliegenden Erfindung liegt die Aufgabe zugrunde, einfacher und rationeller verwendbare lichtempfindliche Zusammensetzungen hoher Empfindlichkeit zur Verfügung zu stellen, die Reliefbilder von höchstem Auflösungsgrad liefern.

Es wurde nun gefunden, daß man lichtempfindliche Zusammensetzungen hoher Empfindlichkeit für fernes UV-Licht, mit deren Hilfe stabile randscharfe Reliefbilder mit ausgezeichneten mechanischen Eigenschaften gebildet werden können, erhält, wenn man dazu lichtempfindliche Zusammensetzungen verwendet, die Bisazidoverbindungen enthalten, bei denen die aromatischen Kerne, welche die Azidogruppen tragen, durch eine verhältnismäßig lange, mehr oder weniger bewegliche Kette und/oder flexible Ringsysteme miteinander verknüpft sind. Durch die besonderen Eigenschaften der erfindungsgemäßen Bisazidoverbindungen ist ihre vernetzende Wirkung auf polymere Verbindungen, welche durch die fotochemische Reaktion ausgelöst wird, besonders effektiv. Der hohe Vernetzungsgrad in den belichteten Bereichen der lichtempfindlichen Zusammensetzungen, die die erfindungsgemäßen Bisazidoverbindungen enthalten, ergibt die hervorragenden mechanischen Eigenschaften der nach dem Entwickeln erhaltenen Reliefbilder.

So weisen die erzeugten Reliefbilder eine ausgezeichnete Haftfestigkeit auf allen in der Halbleiterfertigung üblichen Materialien auf und zeigen hervorragende Resistenz gegen saure und alkalische Ätzmedien. Mit Hilfe der erfindungsgemäßen lichtempfindlichen Zusammensetzungen wird ein hoher Auflösungsgrad sowie ein scharfes Kantenprofil erreicht.

Gegenstand der Erfindung sind Bisazidoverbindungen der allgemeinen Formel I,

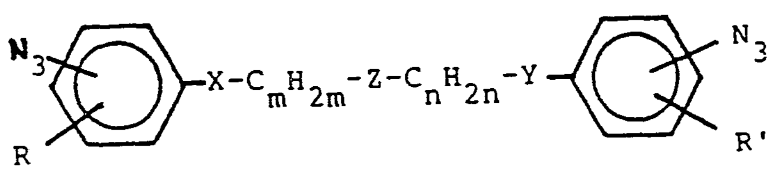

I,

worin

X und Y — die gleich oder verschieden sein können, O, S, $SO_2$, $N\text{-}R_1$ oder eine Bindung,

m und n — die gleich oder verschieden sein können, ganze Zahlen von 1 bis 8, mit der Maßgabe, daß die Summe von m + n nicht größer als 12 ist,

Z — eine Bindung oder, falls X und Y beide eine Bindung bedeuten oder m und n beide ungleich 1 sind, auch O, S, $SO_2$ oder $N\text{-}R_1$, oder, falls

X und Y beide eine Bindung bedeuten, auch CO, gegebenenfalls in 2-Stellung durch eine Oxogruppe substituiertes 1,3-Cyclopentylen oder gegebenenfalls in 2-Stellung durch eine Oxogruppe und/oder in 5-Stellung durch Alkyl mit 1 bis 6 C-Atomen substituiertes 1,3-Cyclohexylen,

R und R' die gleich oder verschieden sein können, H, Halogen oder Alkyl mit 1 bis 6 C-Atomen und

$R_1$ H oder Alkyl mit 1 bis 6 C-Atomen

bedeuten,

mit der Maßgabe, daß $-X-C_mH_{2m}-Z-C_nH_{2n}-Y-$ nicht $-(CH_2)_2-$ oder $-(CH_2)_3-$ bedeutet.

Gegenstand der Erfindung sind ferner negativ arbeitende, für fernes UV-Licht empfindliche Zusammensetzungen, die dadurch gekennzeichnet sind, daß als lichtempfindliche Komponenten mindestens eine Bisazidoverbindung der allgemeinen Formel I

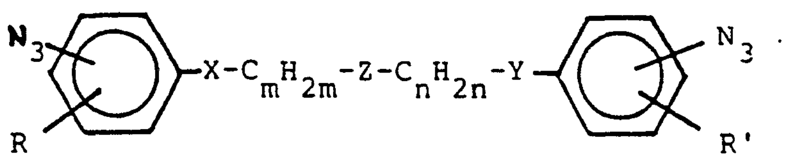

I,

worin

X und Y die gleich oder verschieden sein können, O, S, $SO_2$, $N-R_1$ oder eine Bindung,

m und n die gleich oder verschieden sein können, ganze Zahlen von 1 bis 8, mit der Maßgabe, daß die Summe von m + n nicht größer als 12 ist,

Z eine Bindung oder, falls X und Y beide eine Bindung oder m und n beide ungleich 1 sind, auch O, S, $SO_2$ oder $N-R_1$, oder, falls X und

Y beide eine Bindung bedeuten, auch CO, gegebenenfalls in 2-Stellung durch eine Oxogruppe substituiertes 1,3-Cyclopentylen oder gegebenenfalls in 2-Stellung durch eine Oxogruppe und/oder in 5-Stellung durch Alkyl mit 1 bis 6 C-Atomen substituiertes 1,3-Cyclohexylen,

R und R' die gleich oder verschieden sein können, H, Halogen oder Alkyl mit 1 bis 6 C-Atomen und

$R_1$ H oder Alkyl mit 1 bis 6 C-Atomen

bedeuten,
mit der Maßgabe, daß $-X-C_mH_{2m}-Z-C_nH_{2n}-Y-$ nicht $-(CH_2)_2-$ oder $-(CH_2)_3-$ bedeutet, enthalten ist.

Gegenstand der Erfindung ist weiterhin die Verwendung dieser Bisazidoverbindungen als lichtempfindliche Komponente in negativ arbeitenden, für fernes UV-Licht empfindlichen Zusammensetzungen zum Erzeugen von Reliefstrukturen.

Gegenstand der Erfindung ist auch ein Verfahren zur fotolithographischen Herstellung von Reliefstrukturen durch Auftragen von lichtempfindlichen Zusammensetzungen in Form einer Schicht oder Folie auf ein Substrat, Bestrahlen der lichtempfindlichen Schicht durch eine Maske und Herauslösen oder Abziehen der nicht bestrahlten Schicht- oder Folienteile, das dadurch gekennzeichnet ist, daß man eine erfindungsgemäße negativ arbeitende, für fernes UV-Licht empfindliche Zusammensetzung verwendet und mit ferner UV-Strahlung belichtet.

Die erfindungsgemäßen Bisazidoverbindungen der Formel I umfassen bevorzugt symmetrische Bisazidoverbindungen wie beispielsweise Azidoaryloxyalkoxyarylazide der Formel Ia, Azidoarylthioalkylthioarylazide der Formel Ib, Azidoaryl-sulfonylalkylsulfonyl-arylazide der

Formel Ic, Azidoarylamino-alkylaminoarylazide der Formel Id, Azidoarylalkyl-arylazide der Formel Ie, Azidoarylalkoxyalkyl-arylazide der Formel If, Azidoaryl-alkylthioalkyl-arylazide der Formel Ig, Azidoarylalkylsulfonylalkyl-arylazide der Formel Ih, Azidoarylalkylaminoalkyl-arylazide der Formel Ii, Azidoaryloxy-alkoxyalkoxyarylazide der Formel Ij, Azidoarylthio-alkoxyalkylthioarylazide der Formel Ik, Azidoarylthio-alkylthioalkylthio-arylazide der Formel Il, Azidoaryl-aminoalkoxyalkylamino-arylazide der Formel Im, Azidoarylaminoalkylsulfonylalkylamino-arylazide der Formel In, Azidoarylsulfonylalkylsulfonylalkylsulfonyl-arylazide der Formel Io, Azidoaryloxyalkylaminoalkoxy-arylazide der Formel Ip, Azidoaryloxyalkylsulfonylalkoxy-arylazide der Formel Iq, Bis(azidoarylalkyl)ketone der Formel Ir, 1,3-Bis(azidoarylalkyl)cyclopentane der Formel Is, 2,5-Bis(azidoarylalkyl)cyclopentanone der Formel It, 1,3-Bis(azidoarylalkyl)cyclohexane der Formel Iu, 2,6-Bis(azidoarylalkyl)cyclohexanone der Formel Iv

| | | |
|---|---|---|
| $N_3\text{-Ar-O-}C_mH_{2m}\text{-}C_nH_{2n}\text{-O-Ar-}N_3$ | | Ia |
| $N_3\text{-Ar-S-}C_mH_{2m}\text{-}C_nH_{2n}\text{-S-Ar-}N_3$ | | Ib |
| $N_3\text{-Ar-}SO_2\text{-}C_mH_{2m}\text{-}C_nH_{2n}\text{-}SO_2\text{-Ar-}N_3$ | | Ic |
| $N_3\text{-Ar-}NR_1\text{-}C_mH_{2m}\text{-}C_nH_{2n}\text{-}NR_1\text{-Ar-}N_3$ | | Id |
| $N_3\text{-Ar-}C_mH_{2m}\text{-}C_nH_{2n}\text{-Ar-}N_3$ | $m + n \geq 4$ | Ie |
| $N_3\text{-Ar-}C_mH_{2m}\text{-O-}C_nH_{2n}\text{-Ar-}N_3$ | $m = n$ | If |
| $N_3\text{-Ar-}C_mH_{2m}\text{-S-}C_nH_{2n}\text{-Ar-}N_3$ | $m = n$ | Ig |
| $N_3\text{-Ar-}C_mH_{2m}\text{-}SO_2\text{-}C_mH_{2m}\text{-Ar-}N_3$ | $m = n$ | Ih |
| $N_3\text{-Ar-}C_mH_{2m}\text{-}NR_1\text{-}C_nH_{2n}\text{-Ar-}N_3$ | $m = n$ | Ii |
| $N_3\text{-Ar-O-}C_mH_{2m}\text{-O-}C_nH_{2n}\text{-O-Ar-}N_3$ | $m = n \geq 2$ | Ij |
| $N_3\text{-Ar-S-}C_mH_{2m}\text{-O-}C_nH_{2n}\text{-S-Ar-}N_3$ | $m = n \geq 2$ | Ik |
| $N_3\text{-Ar-S-}C_mH_{2m}\text{-S-}C_nH_{2n}\text{-S-Ar-}N_3$ | $m = n \geq 2$ | Il |
| $N_3\text{-Ar-}NR_1\text{-}C_mH_{2m}\text{-O-}C_nH_{2n}\text{-}NR_1\text{-Ar-}N_3$ | $m = n \geq 2$ | Im |
| $N_3\text{-Ar-}NR_1\text{-}C_mH_{2m}\text{-}SO_2\text{-}C_nH_{2n}\text{-}NR_1\text{-Ar-}N_3$ | $m= n \geq 2$ | In |
| $N_3\text{-Ar-}SO_2\text{-}C_mH_{2m}\text{-}SO_2\text{-}C_nH_{2n}\text{-}SO_2\text{-Ar-}N_3$ | $m= n \geq 2$ | Io |
| $N_3\text{-Ar-O-}C_mH_{2m}\text{-}NR_1\text{-}C_nH_{2n}\text{-O-Ar-}N_3$ | $m = n \geq 2$ | Ip |

$N_3-Ar-O-C_mH_{2m}-SO_2-C_nH_{2n}-O-Ar-N_3$ $m = n \geq 2$ Iq

$N_3-Ar-C_mH_{2m}-CO-C_nH_{2n}-Ar-N_3$ $m = n$ Ir

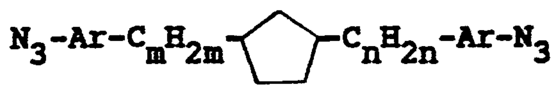

$m = n$ Is

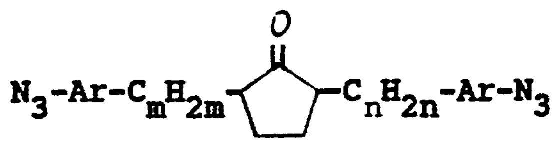

$m = n$ It

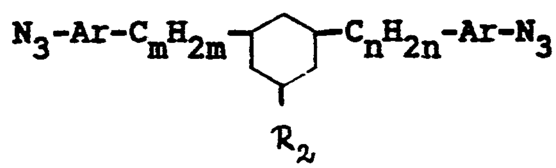

$m = n$ Iu

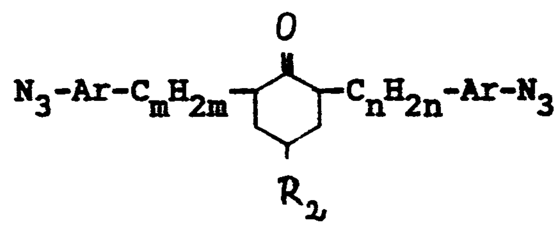

$m = n$ Iv

worin Ar einen Phenylen-rest oder substituierten Phenylen-rest, $-NR_1-$ eine Amino-gruppe -NH- oder eine substituierte Amino-gruppe und $R_2$ Wasserstoff oder eine Alkylgruppe mit 1 bis 6 C-Atomen bedeutet.

Weiterhin umschließt die Formel I asymmetrische Bisazidoverbindungen wie beispielsweise

Azidoarylalkoxyalkylarylazide der Formel I aa,
Azidoarylalkylthioalkylarylazide der Formel I bb,
Azidoarylalkylsulfonylalkylarylazide der Formel I cc,
Azidoarylalkylaminoalkylarylazide der Formel I dd,
Azidoarylalkoxyarylazide der Formel I ee,
Azidoarylalkylaminoarylazide der Formel I ff

Azidoarylalkylthioarylazide der Formel I gg,
Azidoarylalkylsulfonylarylazide der Formel I hh,
Azidoaryloxyalkylthioarylazide der Formel I ii,
Azidoaryloxyalkylsulfonylarylazide der Formel I jj,
Azidoaryloxyalkylaminoarylazide der Formel I kk,
Azidoarylthioalkylsulfonylarylazide der Formel I ll,
Azidoarylthioalkylaminoarylazide der Formel I mm,
Azidoarylalkoxyalkylaminoarylazide der Formel I nn,
Azidoaryloxyalkoxyalkoxyarylazide der Formel I oo,
Azidoaryloxyalkylaminoalkoxyarylazide der Formel I pp,
Bis(azidoarylalkyl)ketone der Formel I qq,
1,3-Bis(azidoarylalkyl)cyclopentane der Formel I rr,
2,5-Bis(azidoarylalkyl)cyclopentanone der Formel I ss,
1,3-Bis(azidoarylalkyl)cyclohexane der Formel I tt,
2,6-Bis(azidoarylalkyl)cyclohexanone der Formel I uu

| | | |
|---|---|---|
| $N_3$-Ar-$C_mH_{2m}$-O-$C_nH_{2n}$-Ar-$N_3$ | $m \neq n$ | I aa |
| $N_3$-Ar-$C_mH_{2m}$-S-$C_nH_{2n}$-Ar-$N_3$ | $m \neq n$ | I bb |
| $N_3$-Ar-$C_mH_{2m}$-$SO_2$-$C_nH_{2n}$-Ar-$N_3$ | $m \neq n$ | I cc |
| $N_3$-Ar-$C_mH_{2m}$-$NR_1$-$C_nH_{2n}$-Ar-$N_3$ | $m \neq n$ | I dd |
| $N_3$-Ar-$C_mH_{2m}$-$C_nH_{2n}$-O-Ar-$N_3$ | | I ee |
| $N_3$-Ar-$C_mH_{2m}$-$C_nH_{2n}$-$NR_1$-Ar-$N_3$ | | I ff |
| $N_3$-Ar-$C_mH_{2m}$-$C_nH_{2n}$-S-Ar-$N_3$ | | I gg |
| $N_3$-Ar-$C_mH_{2m}$-$C_nH_{2n}$-$SO_2$-Ar-$N_3$ | | I hh |
| $N_3$-Ar-O-$C_mH_{2m}$-$C_nH_{2n}$-S-Ar-$N_3$ | | I ii |
| $N_3$-Ar-O-$C_mH_{2m}$-$C_nH_{2n}$-$SO_2$-Ar-$N_3$ | | I jj |
| $N_3$-Ar-O-$C_mH_{2m}$-$C_nH_{2n}$-$NR_1$-Ar-$N_3$ | | I kk |
| $N_3$-Ar-S-$C_mH_{2m}$-$C_nH_{2n}$-$SO_2$-Ar-$N_3$ | | I ll |
| $N_3$-Ar-S-$C_mH_{2m}$-$C_nH_{2n}$-$NR_1$-Ar-$N_3$ | | I mm |
| $N_3$-Ar-$C_mH_{2m}$-O-$C_nH_{2n}$-$NR_1$-Ar-$N_3$ | | I nn |
| $N_3$-Ar-O-$C_mH_{2m}$-O-$C_nH_{2n}$-O-Ar-$N_3$ | $m \neq n$ | I oo |
| $N_3$-Ar-O-$C_mH_{2m}$-$NR_1$-$C_nH_{2n}$-O-Ar-$N_3$ | $m \neq n$ | I pp |
| $N_3$-Ar-$C_mH_{2m}$-CO-$C_nH_{2n}$-Ar-$N_3$ | $m \neq n$ | I qq |

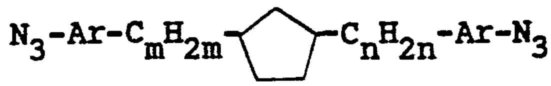

$m \neq n$ I rr

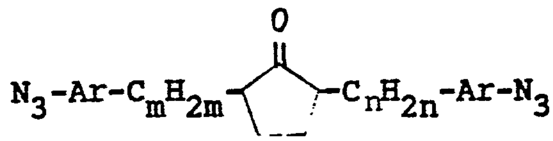

$m \neq n$ I ss

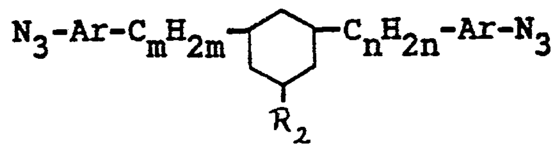

$m \neq n$ I tt

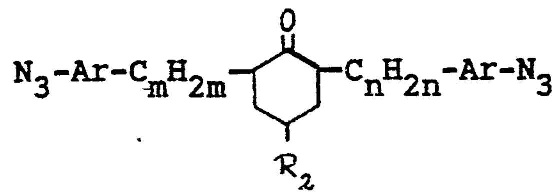

$m \neq n$ I uu

In den Verbindungen der Formel I sind die Alkylen-reste $-C_mH_{2m}-$ bzw. $-C_nH_{2n}-$ vorzugsweise geradkettig, bedeuten also vorzugsweise Methylen, mit der Einschränkung, daß Methylen auf der einen Seite direkt mit einem C-Atom verknüpft sein muß, Ethylen, Propylen, Butylen, Pentylen, Hexylen, Heptylen oder Oktylen.

Sie können jedoch auch verzweigt-kettig sein, wie beispielsweise 1-Methyl-ethylen, 2-Methyl-propylen, 2-Ethyl-butylen, 3-Ethyl-hexylen.

R und R' sind vorzugsweise beide Wasserstoff. Aber auch Verbindungen der Formel I mit substituierten Phenylenresten können gelegentlich wegen besserer oder spezifischerer Lichtempfindlichkeit von Bedeutung sein. In solchen Fällen sind die Substituenten R und R' gewöhnlich Halogenatome Chlor, Brom oder Jod oder stehen an Stelle von Methyl-, Ethyl-, Propyl-, Isopropyl-, Butyl-, Isobutyl-, t-Butyl-, Pentyl- oder Hexyl-gruppen.

$R_1$ bedeutet Wasserstoff oder Alkyl mit 1 bis 6 C-Atomen, wie vorzugsweise Methyl, Ethyl, Propyl, Isopropyl, aber auch beispielsweise Butyl, Isobutyl, tert. Butyl, Pentyl oder Hexyl.

Die Azidogruppe befindet sich vorzugsweise in 4- oder 3-Stellung, kann aber auch in 2-Stellung an den Phenylring gebunden sein. Falls R und/oder R' ungleich Wasserstoff sind, befinden sich diese Substituenten vorzugsweise in ortho-Stellung zur Azidogruppe, sie können aber auch meta- oder para-ständig (sollte die Azidogruppe in 3-Stellung sein) zur Azidogruppe sein. Besonders bevorzugt sind Bisazidoverbindungen, in denen beide Phenylringe in gleicher Art substituiert sind.

Bevorzugte Verbindungen aus dem Bereich der Formeln Ia bis Iv sind:

1,2-Bis(4-azidophenoxy)-ethan
1,2-Bis(3-azidophenoxy)-ethan
1,3-Bis(4-azidophenoxy)-propan
1,3-Bis(3-azidophenoxy)-propan
1,4-Bis(4-azidophenoxy)-butan
1,4-Bis(3-azidophenoxy)-butan
1,2-Bis(4-azidophenylthio)-ethan
1,2-Bis(3-azidophenylthio)-ethan
1,3-Bis(4-azidophenylthio)-propan
1,3-Bis(3-azidophenylthio)-propan
1,4-Bis(4-azidophenylthio)-butan
1,4-Bis(3-azidophenylthio)-butan
1,2-Bis(4-azidophenylsulfonyl)-ethan
1,3-Bis(4-azidophenylsulfonyl)-propan
1,4-Bis(4-azidophenylsulfonyl)-butan
1,4-Bis(3-azidophenylsulfonyl)-butan
1,2-Bis(4-azidophenylamino)-ethan

1,2-Bis[N-(4-azidophenyl)-N-methyl-amino]-ethan
1,4-Bis(4-azidophenyl)-butan
1,5-Bis(4-azidophenyl)-pentan
1,6-Bis(4-azidophenyl)-hexan
1,5-Bis(4-azidophenyl)-3-oxapentan
1,5-Bis(4-azidophenyl)-3-thiapentan
1,5-Bis(4-azidophenyl)-3-thiapentan-3,3-dioxid
1,5-Bis(4-azidophenyl)-3-azapentan
1,5-Bis(4-azidophenoxy)-3-oxapentan
1,5-Bis(4-azidophenylthio)-3-oxapentan
1,5-Bis(4-azidophenylthio)-3-thiapentan
1,5-Bis(4-azidophenylamino)-3-oxapentan
1,5-Bis(4-azidophenylamino)-3-thiapentan-3,3-dioxid
1,5-Bis(4-azidophenylsulfonyl)-3-thiapentan-3,3-dioxid
1,5-Bis(4-azidophenoxy)-3-methyl-3-azapentan
1,5-Bis(4-azidophenoxy)-3-ethyl-3-azapentan
1,5-Bis(4-azido-4-chlor-phenoxy)-3-methyl-3-azapentan
1,5-Bis(4-azidophenoxy)-3-thiapentan-3,3-dioxid
1,5-Bis(3-azido-4-tolyloxy)-3-thiapentan-3,3-dioxid
1,4-Bis(3-azido-4-tolyl)-butan
1,3-Bis(4-azidophenyl)-propan-2-on
1,5-Bis(4-azidophenyl)-pentan-3-on
1,7-Bis(4-azidophenyl)-heptan-4-on
1,9-Bis(4-azidophenyl)-nonan-5-on
1,5-Bis(3-azido-4-tolyl)-3-oxapentan
1,5-Bis(4-azido-6-chlorphenyl)-3-oxapentan
2,6-Bis(4-azidophenylmethyl)-cyclohexanon
2,6-Bis(4-azidophenylmethyl)-4-methyl-cyclohexanon
2,6-Bis(4-azidophenylmethyl)-4-ethyl-cyclohexanon
2,6-Bis[3-(4-azidophenyl)propyl]-cyclohexanon
2,6-Bis[3-(4-azidophenyl)propyl]-4-methyl-cyclohexanon
1,3-Bis(4-azidophenylmethyl)-cyclohexan
1,3-Bis(4-azidophenylmethyl)-5-methyl-cyclohexan
1,3-Bis(4-azidophenylmethyl)-5-ethyl-cyclohexan
1,3-Bis[3-(4-azidophenyl)propyl]-cyclohexan
1,3-Bis[3-(4-azidophenyl)propyl]-5-methyl-cyclohexan

Die Verbindungen der Formel I werden nach an sich bekannten Methoden hergestellt, wie sie in der Literatur (beispielsweise in den Standardwerken wie Houben-Weyl, Methoden der organischen Chemie, Georg Thieme Verlag Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können teilweise auch in situ gebildet werden, derart, daß man sie aus dem Reaktionsgemisch nicht isoliert, sondern weiter zu den Verbindungen der Formel I umsetzt.

Die Endstufen zur Herstellung der Bisazidoverbindungen der Formel I sind gewöhnlich dadurch gekennzeichnet, daß man die entsprechenden Bisamino-verbindungen diazotiert und die erhaltenen Bisdiazoniumverbindungen in an sich bekannter Weise mit Aziden, gewöhnlich Alkaliaziden, umsetzt gemäß:

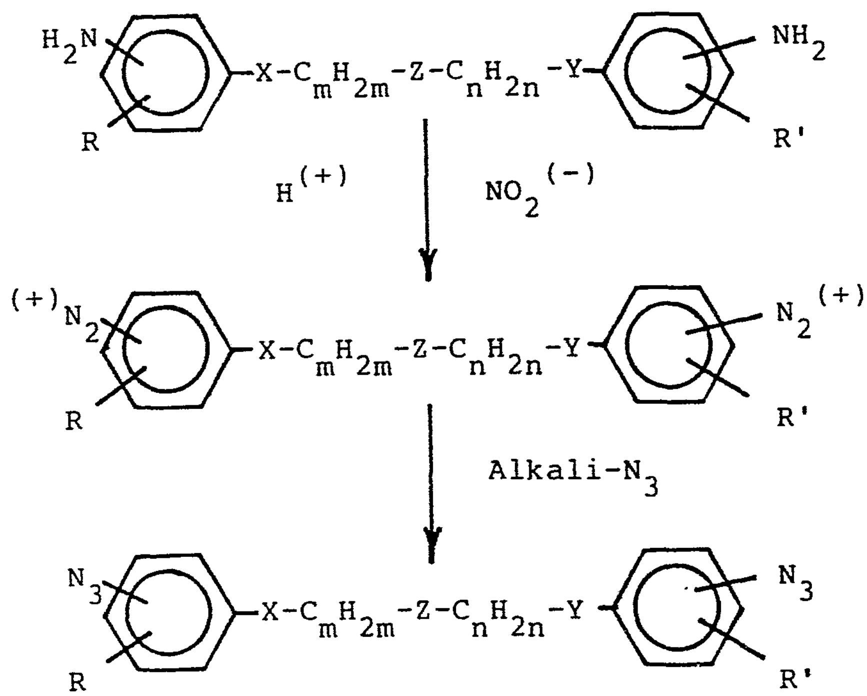


Dabei wird die Diazotierung zweckmäßig mit Natriumnitrit in wäßriger Mineralsäure durchgeführt.

Die als Ausgangsstoffe erforderlichen Bisaminoverbindungen können beispielsweise folgendermaßen hergestellt werden: Zur Herstellung von Verbindungen mit X, Y = O, S oder $NR_1$ werden Aminophenole, Aminothiophenole, Aminoaniline oder Amino-N-alkylaniline, in denen jeweils die Aminogruppe geschützt vorliegt, durch Umsetzung mit bifunktionellen Halogenverbindungen der Formel Hal-$C_mH_{2m}$-Z-$C_nH_{2n}$-Hal alkyliert. Als Schutzgruppen eignen sich alle bekannten Amino-Schutzgruppen, wie beispielsweise ein Acylrest, zweckmäßig der Acetylrest, oder der Benzylrest. Diese Alkylierung wird in der Regel bei Temperaturen zwischen 20 und 180°,

vorzugsweise zwischen Raumtemperatur und der Siedetemperatur des verwendeten Lösungsmittels, in einem inerten Lösungsmittel, wie beispielsweise einem Alkohol, einem Kohlenwasserstoff (zweckmäßig Benzol, Toluol), einem Amid wie Dimethylformamid, Dimethylacetamid oder Hexamethylphosphorsäuretriamid, in Gegenwart eines basischen Katalysators durchgeführt. Als basische Katalysatoren kommen beispielsweise Alkalicarbonate, -hydrogencarbonate, Alkali- oder Erdalkalialkoholate oder Alkalihydride wie Natriumhydrid infrage.

Weiterhin können aber auch alle anderen zur Alkylierung von Phenolen, Thiophenolen oder Anilinen bekannten Verfahren verwendet werden.

Verbindungen mit X, Y und/oder Z = $SO_2$ können durch Oxidation der entsprechenden Bisaminoverbindungen mit X, Y und/oder Z = S, in denen die Aminogruppe geschützt oder ungeschützt vorliegen kann, mit den hierfür bekannten Oxidationsmitteln hergestellt werden. Als Oxidationsmittel eignen sich insbesondere $H_2O_2$ und organische Persäuren wie Peressigsäure, Perbenzoesäure oder substituierte Perbenzoesäuren wie beispielsweise m-Chlorperbenzoesäure. Man arbeitet zweckmäßig in einem inerten organischen Lösungsmittel wie beispielsweise Dichlormethan. Die Oxidation der -S- zur $SO_2$-Gruppe kann aber auch in einer späteren Stufe der Synthese erfolgen.

Diese Bisaminoverbindungen, in denen die Aminogruppe geschützt vorliegt, werden anschließend unter Abspaltung der jeweiligen Schutzgruppen in die entsprechenden freien Bisaminoverbindungen überführt. Die Art der Abspaltung dieser Schutzgruppen richtet sich nach deren chemischer Natur und ist allgemein bekannt. So können beispielsweise Acylreste wie der Acetylrest durch saure oder alkalische Hydrolyse in wäßrigem oder alkoholischem Medium bei Temperaturen von 50 bis 200$^{o}$ gegebenenfalls unter Druck abgespalten werden. Der Benzylrest kann zweckmäßig durch katalytische Hydrierung abgespalten werden.

Die als Ausgangsstoffe erforderlichen Bisaminoverbindungen können auch aus den entsprechenden Bis-Nitroverbindungen durch Reduktion erhalten werden. Zur Reduktion kommen die hierfür bekannten Verfahren in Betracht, wie beispielsweise katalytische Hydrierung, Reduktion mit Eisen oder Zink in mineralsaurer, vorzugsweise salzsaurer Lösung, mit Schwefelwasserstoff sowie weitere bekannte Verfahren zur Überführung einer Nitro- in eine Aminogruppe.

Die Bis-Nitroverbindungen ihrerseits können durch Nitrierung von entsprechenden Bis-aryl-verbindungen, die wiederum durch Alkylierung von Phenolen, Thiophenolen, Anilinen oder N-Alkylanilinen mit bifunktionellen Halogenverbindungen $Hal-C_mH_{2m}-Z-C_nH_{2n}-Hal$ unter den bereits erwähnten Reaktionsbedingungen zugänglich sind, erhalten oder aus Nitrophenolen, Nitrothiophenolen, Nitroanilinen oder Nitro-N-alkyl-anilinen aufgebaut werden. Die Verknüpfung der beiden Nitro-arylreste über die Brücke $-X-C_mH_{2m}-Z-C_nH_{2n}-Y-$ erfolgt nach den klassischen Methoden der organischen Chemie. Beispielsweise werden Nitrophenole, Nitrothiophenole, Nitroaniline oder Nitro-N-alkyl-aniline mit bifunktionellen Halogenverbindungen vom Typus $Hal-C_mH_{2m}-Z-C_nH_{2n}-Hal$ umgesetzt. In einigen Fällen können auch Azidophenole, Azidothiophenole, Azidoaniline oder Azido-N-alkylaniline selbst mit bifunktionellen Halogenverbindungen vom Typus Hal-$C_mH_{2m}$-Z-$C_nH_{2n}$-Hal direkt zu den gewünschten Bisazidoverbindungen der Formel I umgesetzt werden.

Auch bei diesen Herstellungsverfahren können Verbindungen mit X, Y = $SO_2$ durch Oxidation aus den entsprechenden Verbindungen mit X, Y = S erhalten werden.

Verbindungen mit X, Y = -N-Alkyl- können ferner durch Alkylierung von Verbindungen mit X, Y = -NH- mit Hilfe der hierfür bekannten Alkylierungsmittel, wie beispielsweise Alkylhalogeniden, Alkylsulfaten, unter den ebenfalls geläufigen Reaktionsbedingungen erhalten werden.

Verbindungen der Formel I, in denen X, Y = eine Bindung bedeutet, können beispielsweise folgendermaßen hergestellt werden: Aniline, in denen die Aminogruppe durch eine der üblichen Schutzgruppen geschützt vorliegt, werden mittels Friedel-Crafts-Acylierungen mit Dicarbonsäurechloriden der Formel Cl-CO-$C_mH_{2m}$-Z-$C_nH_{2n}$-CO-Cl zu den entsprechenden Diketo-Verbindungen umgesetzt. Man arbeitet dabei unter den für Friedel-Crafts-Acylierungen üblichen Reaktionsbedingungen, in Gegenwart einer Lewis-Säure, wie beispielsweise Aluminiumtrichlorid, gegebenenfalls in einem Lösungsmittel, das die Reaktivität der Lewis-Säure nicht beeinträchtigt. Als Lösungsmittel eignen sich beispielsweise Schwefelkohlenstoff, aromatische Kohlenwasserstoffe wie Nitrobenzol oder bei Reaktionstemperaturen bis zu 50$^o$ auch Halogenkohlenwasserstoffe wie Dichlorethan oder Trichlorethylen.

Die Carbonylgruppen in den erhaltenen Diketoverbindungen werden anschließend zu -$CH_2$- -Gruppen reduziert, zweck-

mäßig nach Clemmensen (beispielsweise mit amalgamiertem Zink/Salzsäure) oder nach Wolff-Kishner (beispielsweise Hydrazin/Kaliumhydroxid in Diethylenglycol). Danach werden die Amino-Schutzgruppen in üblicher Weise abgespalten und die erhaltenen Bis-Aminoverbindungen wie bereits erwähnt diazotiert und mit einem Azid zu den Verbindungen der Formel I umgesetzt.

Eine andere Möglichkeit zur Synthese der Verbindungen der Formel I, in denen X, Y = eine Bindung bedeuten, besteht darin, daß man in beiden aromatischen Ringen von α,ω-Diarylalkanen, α,ω-Diarylheteroalkanen oder α,ω-Diarylalkanonen durch Nitrierung jeweils eine Nitrogruppe einführt, die Nitrogruppen zu den Aminogruppen reduziert, diese diazotiert und die erhaltene Bis-diazonium-Verbindung mit einem Azid zur gewünschten Bisazidoverbindung umsetzt. Dabei arbeitet man in den einzelnen Reaktionsstufen zweckmäßig nach den bereits für solche Umsetzungen erwähnten Reaktionsbedingungen.

Nach diesen Reaktionsfolgen werden bevorzugt Bisazidoverbindungen hergestellt, in denen die Azidogruppen in 4-Stellung an den Phenylring gebunden sind.

Zur Herstellung von Biazidoverbindungen der Formel I mit X, Y = eine Bindung, in denen die Azidogruppen in 3-Stellung an den Phenylring gebunden sind, geht man zweckmäßig von Diketoverbindungen der Formel 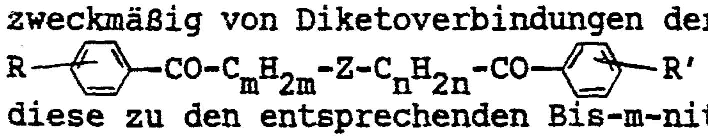 aus, nitriert diese zu den entsprechenden Bis-m-nitroverbindungen, reduziert die Nitrogruppen sowie die Carbonylgruppen gleichzeitig oder stufenweise unter den bereits genannten Reaktionsbedingungen zu den entsprechenden Bis-aminoverbindungen, die dann in üblicher Weise durch Diazotierung und Umsetzung mit Aziden zu Bisazidoverbindungen der Formel I umgesetzt werden.

Bisazidoverbindungen der Formel I, in denen X, Y = eine Bindung und Z = in 2-Stellung durch eine Oxogruppe substituiertes 1,3-Cyclopentylen oder in 2-Stellung durch eine Oxogruppe und gegebenenfalls in 5-Stellung durch Alkyl mit 1 bis 6 C-Atomen substituiertes 1,3-Cyclohexylen bedeuten, können beispielsweise hergestellt werden durch katalytische Hydrierung der entsprechenden ungesättigten Bisazidoverbindungen der Formeln

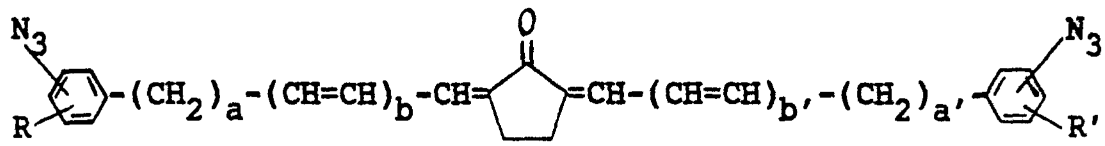

bzw.

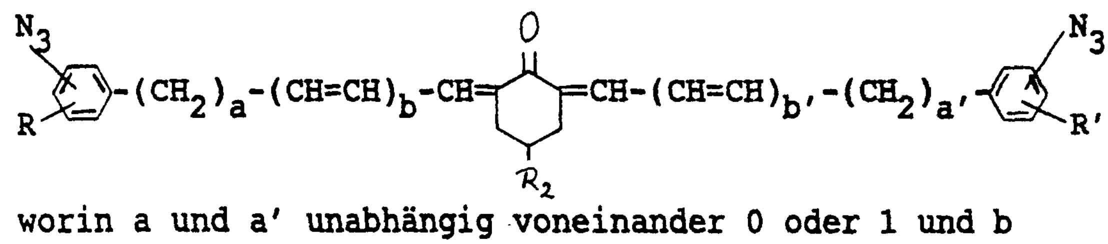

worin a und a' unabhängig voneinander 0 oder 1 und b und b' unabhängig voneinander 0, 1, 2 oder 3 bedeuten, die Summe der C-Atome in beiden Ketten jedoch nicht größer als 12 sein darf. Solche Verbindungen sind bekannt oder können in Analogie zu den bekannten Verbindungen hergestellt werden. Dabei entstehen zunächst die entsprechenden gesättigten Bisaminoverbindungen, die dann in üblicher Weise durch Diazotierung und Umsetzung mit Aziden zu den genannten Bisazidoverbindungen der Formel I umgesetzt werden.

Die vorstehend beschriebenen gesättigten Bisaminoverbindungen können auch zunächst zur Überführung der im Cyclopentanon- bzw. Cyclohexanonsystem vorhandenen Carbonylgruppe in eine $CH_2$-Gruppe nach Wolff-Kishner mit Hydrazin/Kaliumhydroxid oder nach Clemmensen mit amalgamiertem Zink/Salzsäure behandelt werden. Die

hierbei entstehenden Bisaminoverbindungen können dann in üblicher Weise durch Diazotierung und Umsetzung mit Aziden zu Bisazidoverbindungen der Formel I, in denen X, Y = eine Bindung, Z = 1,3-Cyclopentylen oder gegebenenfalls in 5-Stellung mit einer Alkylgruppe mit 1 bis 6 C-Atomen substituiertes 1,3-Cyclohexylen bedeuten, umgesetzt werden.

Die negativ arbeitenden, für fernes UV-Licht empfindlichen Zusammensetzungen gemäß der vorliegenden Erfindung enthalten neben Bisazidoverbindungen der Formel I noch die bei negativ arbeitenden lichtempfindlichen Zusammensetzungen üblichen Bestandteile. Dabei handelt es sich im wesentlichen um mindestens eine polymere Verbindung, die mit den fotochemisch erzeugten Reaktionsprodukten der genannten Bisazidoverbindungen vernetzbar ist, sowie gegebenenfalls weitere, in dieser Technologie bekannte und übliche Zusatzstoffe, die gemeinsam in einem Lösungsmittel oder Lösungsmittelgemisch gelöst sein können.

Die in den erfindungsgemäßen Zusammensetzungen zu verwendenden polymeren Verbindungen weisen im fernen UV-Bereich, insbesondere im Wellenlängenbereich von 250 bis 270 nm keine oder nur eine geringe Absorption auf. Auf diese Weise wird bei Belichtung mit fernem UV-Licht das eingestrahlte Licht selektiv von den in den erfindungsgemäßen Zusammensetzungen enthaltenen Bisazidoverbindungen absorbiert, wodurch die Fotoreaktion ausgelöst wird und die nachfolgende Vernetzungsreaktion abläuft.

Beispiele für geeignete polymere Verbindungen bzw. Reaktionspartner für die Vernetzung sind Naturkautschuk,

cyclisierter Naturkautschuk, Polybutadien, Polyisopren, partiell cyclisiertes cis-1,4-Polyisopren, cyclisiertes Polyisopren, Polychloropren, Nitrilkautschuk, Polyamid, Novolakharze, wie z. B. Kresol-Formaldehyd-Novolake oder Kresol-Alkylphenol-Formaldehyd-Novolake, Polyvinylphenol, Poly-vinylbutyral und andere in der Technik bekannte polymere Verbindungen. Man kann auch Mischungen derartiger polymerer Verbindungen verwenden. Von diesen polymeren Verbindungen sind die kautschukartigen Verbindungen und Novolakharze bevorzugt, da sie Schichten oder Überzüge mit ausgezeichneten Eigenschaften ergeben.

Das Verhältnis von lichtempfindlicher Komponente zu polymerem Reaktionspartner liegt im allgemeinen im Bereich von 1 : 8 bis 1 : 250, vorzugsweise 1 : 15 bis 1 : 100. Als Beispiele für mögliche, in dieser Technologie bekannte und übliche Zusatzstoffe seien Füllstoffe, Fotosensibilisatoren, Farbstoffe, Pigmente, Weichmacher, Haftvermittler, thermisch aktivierbare, freie Radikale bildende Initiatoren genannt, ferner auch die verschiedensten anderen Polymeren und Harze, Stabilisatoren sowie oberflächenaktive Verbindungen, die gegebenenfalls zur Verbesserung der filmbildenden Eigenschaften oder Beschichtungseigenschaften beitragen können und/oder zur Verbesserung der Haftung der auf die Substrate aufgetragenen Schichten, ferner zur Verbesserung der mechanischen Festigkeit, der chemischen Widerstandfähigkeit, der Fließbeständigkeit des Materials, aber auch zur Beeinflussung der Viskosität der lichtempfindlichen Zusammensetzung. Solche Zusatzstoffe können in einer Menge von 0 bis 15 Gew. %, bezogen auf den Feststoffgehalt, zugesetzt werden.

Als Lösungsmittel eignen sich beispielsweise aromatische Kohlenwasserstoffe wie Toluol, Xylol oder Cumol; halogen-substituierte aromatische Kohlenwasserstoffe wie Chlorbenzol und Mischungen solcher Lösungsmittel. Es können aber auch alle anderen Lösungsmittel, die die genannten Bestandteile der lichtempfindlichen Zusammensetzungen zu lösen vermögen, sowie Gemische derartiger Lösungsmittel verwendet werden. Für lichtempfindliche Zusammensetzungen, die polymere Verbindungen vom Typ der Novolakharze enthalten, eignen sich als Lösungsmittel insbesondere Glykolether wie Glykoldimethylether und Glykolmonomethyl- und -ethylether; aliphatische Ester wie Ethylacetat, Hydroxyethylacetat oder Alkoxyethylacetat; Ketone wie Cyclohexanon oder Mischungen solcher Lösungsmittel. Diese Lösungen enthalten in der Regel 5 bis 60 %, vorzugsweise bis 50 % an Feststoffen. Die erfindungsgemäßen lichtempfindlichen Zusammensetzungen können in der üblichen Weise auf alle hierfür gebräuchlichen Substrate aufgetragen werden, insbesondere auf an der Oberfläche thermisch oxidierte und/oder mit Aluminium beschichtete Siliziummaterialien, die gegebenenfalls auch dotiert sein können, ferner alle anderen in der Halbleitertechnologie üblichen Substrate wie z.B. Siliziumnitrid, Galliumarsenid, Indiumphosphid. Weiterhin kommen infrage aus der Flüssigkristalldisplay-Herstellung bekannte Substrate wie Glas, Indium-Zinn-oxid; ferner Metallplatten und -folien, beispielsweise aus Aluminium, Kupfer, Zink; Bimetall- und Trimetallfolien, aber auch elektrisch nicht leitende Folien, die mit Metallen bedampft sind, gegebenenfalls mit Aluminium beschichtete $SiO_2$-Materialien, Papier. Diese Substrate können einer Temperatur-Vorbehandlung unterzogen werden, oberflächlich angerauht, angeätzt oder zur Erhöhung der Hydrophilie mit Chemikalien behandelt sein.

Die lichtempfindlichen Zusammensetzungen können in verschiedener Stärke auf ein Substrat aufgetragen werden. Die im Einzelfalle vorteilhafteste Schichtstärke hängt von verschiedenen Faktoren ab, beispielsweise dem speziellen Verwendungszweck des herzustellenden Materials und der im Einzelfalle verwendeten Bestandteile der lichtempfindlichen Zusammensetzungen. Als zweckmäßig hat es sich in der Regel erwiesen, wenn die Schichten eine Stärke von 0,1 µm bis 10 µm aufweisen.

Der Auftrag der lichtempfindlichen Zusammensetzungen auf die saubere Oberfläche der Substrate kann durch Aufsprühen, Fließbeschichten, Walzen, Schleuderbeschichten und Tauchbeschichten erfolgen, wonach das Lösungsmittel durch Verdampfen entfernt wird, so daß auf der Oberfläche des Substrats eine strahlungsempfindliche Schicht zurückbleibt. Die Entfernung des Lösungsmittels kann gegebenenfalls durch Erhitzen der Schicht auf Temperaturen von bis zu 100$^{o}$ gefördert werden. Anschließend wird die lichtempfindliche Schicht der Strahlung ausgesetzt. Die Bestrahlung oder Belichtung kann durch eine Maskenvorlage durchgeführt werden, es kann aber auch ein gebündelter Strahl der Strahlung über die Oberfläche der strahlungsempfindlichen Schicht geführt werden. Man bestrahlt gegebenenfalls in einer Inertgasatmosphäre. Üblicherweise werden zur Belichtung UV-Lampen verwendet, die im Bereich von 200 bis 300 nm eine hohe Strahlungsintensität aufweisen.

Um zu verhindern, daß UV-Licht mit einer Wellenlänge größer 300 nm die lichtempfindliche Schicht erreicht, wird zwischen die Lichtquelle und die Maskenvorlage bzw. die lichtempfindliche Schicht ein Kaltspiegel geschaltet, der für Strahlung mit einer Wellenlänge

größer als 300 nm durchlässig ist. Demnach enthält das vom Kaltspiegel reflektierte Licht, das zur Belichtung der lichtempfindlichen Schicht verwendet wird, keine Strahlung mit einer Wellenlänge größer 300 nm. In der Schicht wird ein Reliefmuster unter Freilegung von Teilen des Substrats entwickelt, indem die Schicht mit einer Entwicklerlösung behandelt wird, die die nicht bestrahlten Bereiche der lichtempfindlichen Zusammensetzung entfernt, die belichtete und dadurch vernetzte Schicht jedoch nicht zu lösen vermag.

Typische Entwicklerlösungen bestehen beispielsweise aus aromatischen oder aliphatischen Kohlenwasserstoffen wie Toluol, Xylol, Pentan, Hexan, Cyclohexan, Heptan; chlorierten Kohlenwasserstoffen wie Chlorbenzol oder aus Gemischen dieser Stoffe. Lichtempfindliche Zusammensetzungen, die polymere Verbindungen vom Typ der Novolakharze enthalten, werden vorzugsweise mit wäßrig-alkalischen Entwicklerlösungen wie z. B. Lösungen von Natrium- oder Kaliumhydroxid, -phosphat oder -silikat entwickelt. Es können aber auch alle anderen Stoffe verwendet werden, die in der Lage sind, die nicht belichteten Bereiche der lichtempfindlichen Zusammensetzung zu lösen, ohne dabei die belichteten Bereiche anzugreifen. Nach kurzer Bestrahlung sowie Entwickeln, Waschen und Trocknen werden kantenscharfe Reliefstrukturen erhalten.

Die nach dem erfindungsgemäßen Verfahren erhaltenen Reliefbilder zeichnen sich durch eine ausgezeichnete Haftfestigkeit auf dem jeweiligen Substrat, durch hervorragende Resistenz gegen saure und alkalische Ätzmedien sowie hohe Temperaturbeständigkeit aus. Es wird ein ausgezeichneter Auflösungsgrad in der Größenordnung von 0,5 bis 2 µm und ein gutes Kontrastverhältnis erreicht.

Trotz der hohen Empfindlichkeit der erfindungsgemäßen lichtempfindlichen Zusammensetzungen in fernem UV-Bereich lassen sie sich bei Einhaltung einfacher Vorsichtsmaßnahmen unter normalem oder nur leicht filtriertem Tageslicht verarbeiten, ohne Zersetzung oder Schleierbildung befürchten zu müssen.

Die folgenden Beispiele sollen die Erfindung erläutern, ohne sie zu begrenzen.

Angaben über Mischungen (z.B. 1:3) bedeuten jeweils Gewichtsverhältnisse.

Beispiel 1

15,1 g 4-Hydroxyacetanilid und 5 g 1,2-Dichlorethan werden in 100 ml Dimethylformamid gelöst, mit 30,4 g trockenem Kaliumcarbonat versetzt und unter Rühren einige Stunden auf 100° erhitzt. Man kühlt auf 80° ab, setzt weitere 5 g 1,2-Dichlorethan zu und rührt nochmals 20 Stunden bei 80°. Nach dem Abkühlen auf Zimmertemperatur wird die Mischung in 500 ml Wasser eingerührt. Das ausgefallene Produkt wird abfiltiert und getrocknet.
Man erhält in guter Ausbeute 1,2-Bis(4-acetaminophenoxy)-ethan vom Schmelzpunkt 264 bis 266°.

16,4 g 1,2-Bis(4-acetaminophenoxy)-ethan werden in einer Lösung von 28 g Kaliumhydroxid in 400 ml 50 %igem Ethanol suspendiert. Anschließend wird die Mischung in einem Autoklaven 20 Stunden auf 150° erhitzt. Nach dem Abkühlen werden die festen Anteile des Reaktionsgemisches abgetrennt, mit 50 %igem Ethanol gewaschen und getrocknet.
Das erhaltene 1,2-Bis(4-aminophenoxy)-ethan schmilzt bei 177 bis 178°.

4,9 g 1,2-Bis(4-aminophenoxy)-ethan werden in 40 g halbkonzentrierter Salzsäure suspendiert und bei 0 bis 5° mit einer Lösung von 2,8 g Natriumnitrit in 5 ml Wasser diazotiert. Wenn die Diazotierung beendet ist, fügt man tropfenweise eine Lösung von 2,6 g Natriumazid in 10 ml Wasser zur erhaltenen Diazoniumsalz-Lösung. Die dadurch gebildete Bisazidoverbindung fällt aus. Sie wird abfiltriert und aus Ethanol umkristallisiert. Das erhaltene 1,2-Bis(4-azidophenoxy)-ethan schmilzt bei 140 bis 141° unter Zersetzung.

Beispiele 2 bis 48

Auf ähnliche Weise werden durch Diazotierung und Umsetzung der entsprechenden Diazoniumsalzlösungen mit Alkaliaziden erhalten:

2. 1,3-Bis(4-azidophenoxy)-propan
3. 1,4-Bis(4-azidophenoxy)-butan
4. 1,4-Bis(3-azidophenoxy)-butan
5. 1,4-Bis(3-azido-4-tolyloxy)-butan
6. 1,5-Bis(4-azidophenoxy)-pentan
7. 1,3-Bis(4-azidophenoxy)—2-dimethyl-propan
8. 1,6-Bis(4-azidophenoxy)-hexan
9. 1,6-Bis(3-azidophenoxy)-hexan
10. 1,10-Bis(4-azidophenoxy)-decan
11. 1,12-Bis(4-azidophenoxy)-dodecan
12. 1,2-Bis(4-azidophenylthio)-ethan
13. 1,4-Bis(4-azidophenylthio)-butan
14. 1,4-Bis(3-azidophenylthio)-butan
15. 1,4-Bis(3-azido-4-chlorphenylthio)-butan
16. 1,2-Bis(4-azidophenylamino)-ethan
17. 1,4-Bis(4-azidophenyl)-1,4-dimethyl-1,4-diazabutan
18. 1,4-Bis(3-azido-4-bromphenyl)-1,4-dimethyl-1,4-diazabutan

19. 1,6-Bis(4-azidophenyl)-1,6-dimethyl-1,6-diazahexan
20. 1,6-Bis(2-azido-4-jodphenyl)-1,6-dimethyl-1,6-diazahexan
21. 1,5-Bis(4-azidophenoxy)-3-oxapentan
22. 1,5-Bis(4-azido-2-chlorphenoxy)-3-oxapentan
23. 1,5-Bis(4-azido-2-bromphenoxy)-3-oxapentan
24. 1,5-Bis(3-azidophenoxy)-3-oxapentan
25. 1,5-Bis(4-azido-3-tolyloxy)-3-oxapentan
26. 1,5-Bis(2-azido-4-tolyloxy)-3-oxapentan
27. 1,5-Bis(2-azido-4-t-butylphenoxy)-3-oxapentan
28. 1,9-Bis(4-azidophenoxy)-5-oxanonan
29. 1,5-Bis(4-azidophenylthio)-3-oxapentan
30. 1,5-Bis(4-azidophenylthio)-3-thiapentan
31. 1,5-Bis(3-azido-4-tolylthio)-3-thiapentan
32. 1,5-Bis(4-azidophenylamino)-3-oxapentan
33. 1,7-Bis(4-azidophenyl)-1,7-dimethyl-4-oxa-1,7-diazaheptan
34. 1,5-Bis(4-azidophenylamino)-3-thiapentan-3,3-dioxid
35. 1,5-Bis(2-azido-4-tolylamino)-3-thiapentan-3,3-dioxid
36. 1,7-Bis(4-azidophenyl)-1,7-dimethyl-4-thia-1,7-diazaheptan-4,4-dioxid
37. 1,5-Bis(4-azidophenylsulfonyl)-3-thiapentan-3,3-dioxid
38. 1,5-Bis(3-azido-4-chlorphenylsulfonyl)-3-thiapentan-3,3-dioxid
39. 1,5-Bis(4-azidophenoxy)-3-methyl-3-azapentan
40. 1,5-Bis(3-azidophenoxy)-3-ethyl-3-azapentan
41. 1,5-Bis(3-azido-4-tolyloxy)-3-methyl-3-azapentan
42. 1,5-Bis(3-azido-4-chlorphenylthio)-3-methyl-3-azapentan
43. 1,5-Bis(4-azidophenoxy)-3-ethyl-3-azapentan
44. 1,5-Bis(4-azidophenoxy)-3-isopropyl-3-azapentan

45. 1,5-Bis(4-azidophenoxy)-3-butyl-3-azapentan
46. 1,5-Bis(4-azidophenoxy)-3-thiapentan-3,3-dioxid
47. 1,5-Bis(4-azido-3-tolyloxy)-3-thiapentan-3,3-dioxid
48. 1,5-Bis(2-azido-4-bromphenoxy)-3-thiapentan-3,3 dioxid

Beispiel 49

Durch Friedel-Crafts Reaktion von Acetanilid mit Bernsteinsäuredichlorid wird 1,2-Bis(4-acetylaminobenzoyl)-ethan erhalten. Dieses wird nach Clemmensen mit Zink und Salzsäure zum 1,4-Bis(4-acetylaminophenyl)-butan reduziert.

Die Verseifung, Diazotierung und Umsetzung mit Natriumazid erfolgt nach der Methode von Beispiel 1. Man erhält 1,4-Bis(4-azidophenyl)-butan.

Beispiele 50 bis 53

Analog Beispiel 49 können erhalten werden:

50. 1,4-Bis(4-azido-3-tolyl)-butan
51. 1,5-Bis(4-azido-3-tolyl)-3-oxapentan
52. 1,5-Bis(4-azido-3-tolyl)-3-thiapentan
53. 1,5-Bis(4-azidophenyl)-3-methyl-3-azapentan

Beispiel 54

20,1 g 1,3-Diphenyl-propan-2-on werden langsam in 100 ml rauchende Salpetersäure eingetragen. Durch Kühlung wird die Temperatur zwischen 0 und 4° gehalten. Nach 10 Minuten ist die Reaktion beendet. Die

Reaktionsmischung wird auf Eiswasser gegossen. Der dabei entstehende Niederschlag wird abfiltriert und aus Ethanol umkristallisiert. Man erhält 1,3-Bis(4-nitrophenyl)-propan-2-on.

20 g 1,3-Bis(4-nitrophenyl)-propan-2-on werden in 200 ml Methanol suspendiert und an Raney-Nickel hydriert, bis kein Wasserstoff mehr aufgenommen wird. Nach Abtrennen des Katalysators wird das Lösungsmittel abgezogen und der Rückstand von 1,3-Bis(4-aminophenyl)-propan-2-on aus Alkohol umkristallisiert.

Die Diazotierung und Umsetzung mit Natriumazid erfolgt nach der Methode von Beispiel 1. Man erhält 1,3-Bis(4-azidophenyl)-propan-2-on.

Beispiele 55 bis 61

Analog Beispiel 54 werden erhalten:

55. 1,5-Bis(4-azidophenyl)-pentan-3-on
56. 1,7-Bis(4-azidophenyl)-heptan-4-on
57. 1,5-Bis(4-azidophenyl)-pentan
58. 1,6-Bis(4-azidophenyl)-hexan
59. 1,8-Bis(4-azidophenyl)-octan
60. 1,5-Bis(4-azidophenyl)-3-oxapentan
61. 1,5-Bis(4-azidophenyl)-3-thiapentan

Beispiel 62

37 g 2,6-Bis(4-azidophenylmethylen)-4-methylcyclohexanon werden in 400 ml Toluol gelöst und bei 20° an einem Palladium-Kohle-Katalysator hydriert, bis kein Wasserstoff mehr aufgenommen wird. Nach Abtrennen des

Katalysators wird die Reaktionslösung zur Trockne eingeengt, und der verbleibende Rückstand in 50 ml Petrolether aufgenommen und für 10 Minuten erhitzt. Die unlöslichen Anteile werden abgetrennt und unter vermindertem Druck getrocknet. Man erhält 2,6-Bis(4-aminophenylmethyl)-4-methylcyclohexanon.

Die Diazotierung und Umsetzung mit Natriumazid erfolgt nach der Methode von Beispiel 1. Auf diese Weise wird in guter Ausbeute 2,6-Bis(4-azidophenylmethyl)-4-methylcyclohexanon erhalten.

Beispiele 63 bis 67

Analog Beispiel 62 werden erhalten:

63. 2,6-Bis(4-azidophenylmethyl)-cyclohexanon
64. 2,5-Bis(4-azidophenylmethyl)-4-ethyl-cyclohexanon
65. 2,5-Bis(4-azidophenylmethyl)-cyclopentanon
66 2,6-Bis[3-(4-azidophenyl)-propyl]-4-methyl-cyclohexanon
67. 2,6-Bis[3-(4-azidophenyl)-propyl]-cyclohexanon

Beispiel 68

32 g 2,6-Bis(4-aminophenylmethyl)-4-methyl-cyclohexanon werden in 100 ml Diethylenglykol gelöst und nach Zugabe von 19 g 80 %iger Hydrazin-Lösung und 26 g Kaliumhydroxid zum Rückfluß erhitzt. Nach 2 Stunden werden die niedrigsiedenden Anteile abdestilliert und die Sumpftemperatur allmählich auf 195° gesteigert. Nach einer Stunde bei 195° ist die Reaktion beendet. Nach dem Abkühlen wird die Reaktionsmischung auf Wasser gegeben und mehrmals mit Ether extrahiert. Die Etherphasen werden mit unverdünnter Salzsäure und Wasser

gewaschen, getrocknet und der Ether abdestilliert. Der verbleibende Rückstand besteht aus 1,3-Bis(4-aminophenylmethyl)-5-methyl-cyclohexan.

Die Diazotierung und Umsetzung mit Natriumazid erfolgt nach der Methode von Beispiel 1. Man erhält 1,3-Bis(4-azidophenylmethyl)-5-methyl-cyclohexan.

Beispiele 69 bis 73

Analog Beispiel 68 werden hergestellt:

69. 1,3-Bis(4-azidophenylmethyl)-cyclohexan
70. 1,3-Bis(4-azidophenylmethyl)-5-ethyl-cyclohexan
71. 1,3-Bis(4-azidophenylmethyl)-cyclopentan
72. 1,3-Bis[3-(4-azidophenyl)-propyl]-5-methyl-cyclohexan
73. 1,3-Bis[3-(4-azidophenyl)-propyl]-cyclohexan

Beispiel 74

Asymmetrische Bisazidoverbindungen vom Typus der Formeln Iaa bis I uu werden bevorzugt durch vorsichtige Umsetzung von vorgebildeten Azidophenyl-Verbindungen unter Lichtausschluß und Vermeidung von saurem Reaktionsmilieu hergestellt.

4-Azidophenethylbromid wird vorsichtig mit 4-Azidophenol kondensiert, wobei 1-(4-Azidophenethyloxy)-4-azidobenzol erhalten wird.

Beispiele 75 bis 83

In prinzipiell ähnlicher Weise erhält man:

75. 1-(4-Azidophenethylamino)-4-azidobenzol
76. 1-(4-Azidophenethylthio)-4-azidobenzol
77. 4-Azido-N-methyl-N-(ß-(4-azidophenoxy)-ethyl)-anilin
78. 4-Azido-N-methyl-N-(ß-(4-azidobenzyloxy)-ethyl)-anilin
79. 1-(ß-(4-Azidophenoxy)-ethylthio)-4-azidobenzol
80. 1-(ß-(4-Azidophenoxy)-ethylsulfonyl)-4-azidobenzol
81. 4-Azido-N-methyl-N-(ß-(4-azidophenylthio)-ethyl)-anilin
82. 4-Azidobenzyl-4'-azidophenethyl-ether
83. ß-(4-Azidophenoxy)-ethyl-γ-(4-azidophenoxy)-propylether

Beispiel A

Man löst 10 g partiell cyclisiertes cis-1,4-Polyisopren und 0,2 g 1,2-Bis(4-azidophenoxy)-ethan in 100 g Xylol. Die erhaltene lichtempfindliche Lösung wird auf oberflächenoxidierte Siliciumplättchen aufgeschleudert und anschließend getrocknet. Die dadurch erzeugte lichtempfindliche Schicht hat eine Dicke von etwa 1 µm.

Die Plättchen werden unter Stickstoff durch eine Maske aus Chrom mit "fernem UV-Licht" aus einer 500-W-Xenon-Quecksilber-Lampe belichtet. Dabei wird das Licht der Quecksilber-Lampe mittels eines Kaltspiegels derart gefiltert, daß nur Licht des Wellenlängenbereiches von 200 bis 300 nm auf die Plättchen trifft. Nach der Belichtung wird das Reliefbild (Muster) durch Behandlung mit einer Lösung aus Hexan und Xylol (1:1) entwickelt. Dabei wird die lichtempfindliche Schicht an

den unbelichteten Bereichen entfernt. Das erhaltene Reliefbild zeichnet sich durch gute Haftfestigkeit, hervorragende Ätzresistenz und ein hohes Kontrastverhältnis aus. Die Auflösungsgrenze liegt im Bereich von 0,5 bis 2 µm.

Beispiel B bis W

Analog, wie im Beispiel A beschrieben, können die in den Beispielen 2 bis 83 erwähnten Bisazidoverbindungen oder Mischungen davon zusammen mit Naturkautschuk, cyclisiertem Naturkautschuk, Polybutadien, cyclisiertem Polybutadien, Polyisopren, cyclisiertem Polyisopren, Polychloropren, Novolakharzen und anderen polymeren Verbindungen oder Mischungen solcher polymeren Verbindungen verwendet werden.

Typische Lösungen lichtempfindlicher Zusammensetzungen sind:

| | | | |
|---|---|---|---|
| B | 1,4-Bis(4-azidophenylthio)-butan | 0,2 | g |
| | cyclisierter Polyisoprenkautschuk | 10 | g |
| | Xylol | 90 | g |
| C | 1,5-Bis(4-azidophenoxy)-3-oxapentan | 0,3 | g |
| | partiell cyclisiertes cis-1,4-Polyisopren | 10 | g |
| | Toluol | 100 | g |
| D | 1,4-Bis(4-azidophenylsulfonyl)-butan | 0,1 | g |
| | Kresol-Formaldehyd-Novolakharz | 9,9 | g |
| | Methoxyethylacetat | 90 | g |

| | | | |
|---|---|---|---|
| E | 1,5-Bis(4-azidophenylthio)-3-oxapentan | 0,1 | g |
| | partiell cyclisierter Polyisoprenkautschuk | 9,9 | g |
| | Toluol | 90 | g |
| F | 1,5-Bis(4-azidophenylthio)-3-thiapentan | 0,2 | g |
| | partiell cyclisiertes cis-1,4-Polyisopren | 5 | g |
| | Xylol | 95 | g |
| G | 1,2-Bis(4-azidophenylamino)-ethan | 0,3 | g |
| | cyclisiertes cis-1,4-Polyisopren | 10 | g |
| | Toluol | 100 | g |
| H | 1,5-Bis(4-azidophenylamino)-3-thiapentan-3,3-dioxid | 0,1 | g |
| | Butylphenol-Kresol-Formaldehyd-Novolakharz | 12 | g |
| | Glycolmonomethylether | 88 | g |
| I | 1,3-Bis(4-azidophenyl)-propan-2-on | 0,25 | g |
| | cyclisiertes Polybutadien | 9,75 | g |
| | Toluol | 90 | g |
| K | 1,5-Bis(4-azidophenyl)-pentan-3-on | 0,15 | g |
| | partiell cyclisiertes Polyisopren | 9,85 | g |
| | Chlorbenzol | 90 | g |
| L | 1,5-Bis(3-azido-4-tolyloxy)-3-methyl-3-azapentan | 0,3 | g |
| | Polychloropren | 9,7 | g |
| | Toluol | 90 | g |

| | | | |
|---|---|---|---|
| M | 2,6-Bis(4-azidophenylmethyl)-4-methyl-cyclohexanon | 0,25 | g |
| | cyclisiertes Polyisopren | 9,75 | g |
| | Xylol | 90 | g |
| N | 1,3-Bis(4-azidophenylmethyl)-5-methyl-cyclohexan | 0,1 | g |
| | cyclisierter Naturkautschuk | 9,9 | g |
| | Xylol | 90 | g |
| O | 1,4-Bis(4-azidophenyl)-butan | 0,3 | g |
| | cyclisiertes Polybutadien | 9,7 | g |
| | Toluol | 90 | g |
| P | 1,8-Bis(4-azidophenyl)-octan | 0,3 | g |
| | partiell cyclisiertes Polyisopren | 9,7 | g |
| | Toluol | 90 | g |
| Q | 1,5-Bis(3-azidophenyl)-3-oxapentan | 0,2 | g |
| | partiell cyclisiertes Polyisopren | 9,8 | g |
| | Chlorbenzol | 90 | g |
| R | 1,4-Bis(3-azidophenylthio)-butan | 1 | g |
| | Kresol-Formaldehyd-Novolakharz | 9 | g |
| | Methoxyethylacetat und Glycol-monomethylether | je 45 | g |
| S | 4-Azidobenzyl-4'-azidophenethyl-ether | 0,5 | g |
| | cyclisiertes Polyisopren | 9,5 | g |
| | Toluol | 90 | g |
| T | 2,6-Bis[3-(4-azidophenyl)-propyl]-4-methyl-cyclohexanon | 0,4 | g |
| | cyclisierts Polyisopren | 9,6 | g |
| | Xylol/Toluol 1:2 | 90 | g |

| | | | |
|---|---|---|---|
| U | 1,4-Bis(4-azidophenylthio)-butan | | 0,15 g |
| | 1,4-Bis(4-azidophenyloxy)-butan | | 0,15 g |
| | Kresol-t-Butylphenol-Formaldehyd-Novolakharz | | 10 g |
| | Methoxyethylacetat | | 90 g |
| V | 1,4-Bis(4-azidophenylthio)-butan | | 0,2 g |
| | 2,6-Bis(4-azidophenylmethyl)-4-methylcyclohexanon | | 0,2 g |
| | Polychloropren und partiell cyclisiertes Polyisopren | je | 5 g |
| | Toluol | | 90 g |
| W | 1,4-Bis(4-azidophenyl)-butan | | 0,1 g |
| | 1,5-Bis(4-azidophenoxy)-3-oxapentan | | 0,1 g |
| | Naturkautschuk und cyclisiertes Polybutadien | je | 4,9 g |
| | Xylol | | 90 g |

Merck Patent Gesellschaft
mit beschränkter Haftung
Darmstadt

Patentansprüche:

1. Bisazidoverbindungen der allgemeinen Formel I

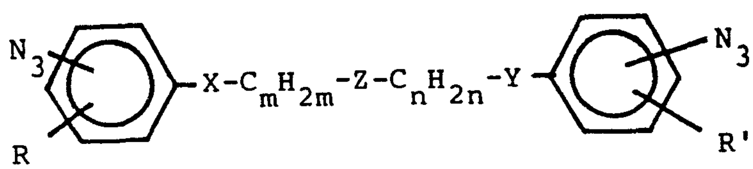

I,

worin

X und Y die gleich oder verschieden sein können, O, S, $SO_2$, $N-R_1$ oder eine Bindung,

m und n die gleich oder verschieden sein können, ganze Zahlen von 1 bis 8, mit der Maßgabe, daß die Summe von m + n nicht größer als 12 ist,

Z eine Bindung oder, falls X und Y beide eine Bindung bedeuten oder m und n beide ungleich 1 sind, auch O, S, $SO_2$ oder $N-R_1$, oder, falls X und Y beide eine Bindung bedeuten, auch CO, gegebenenfalls in 2-Stellung durch eine Oxogruppe substituiertes 1,3-Cyclopentylen oder gegebenenfalls in 2-Stellung durch eine Oxogruppe und/oder in 5-Stellung durch Alkyl mit 1 bis 6 C-Atomen substituiertes 1,3-Cyclohexylen,

R und R' die gleich oder verschieden sein können, H, Halogen oder Alkyl mit 1 bis 6 C-Atomen und

$R_1$ H oder Alkyl mit 1 bis 6 C-Atomen

bedeuten,
mit der Maßgabe, daß $-X-C_mH_{2m}-Z-C_nH_{2n}-Y-$ nicht $-(CH_2)_2-$ oder $-(CH_2)_3-$ bedeutet.

2. Negativ arbeitende, für fernes UV-Licht empfindliche Zusammensetzungen, dadurch gekennzeichnet, daß als lichtempfindliche Komponenten mindestens eine Bisazidoverbindung der allgemeinen Formel I

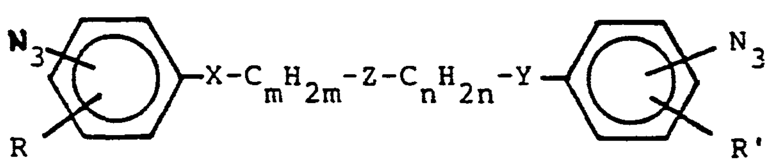

I,

worin

X und Y die gleich oder verschieden sein können, O, S, $SO_2$, $N-R_1$ oder eine Bindung,

m und n die gleich oder verschieden sein können, ganze Zahlen von 1 bis 8, mit der Maßgabe, daß die Summe von m + n nicht größer als 12 ist,

Z eine Bindung, oder falls X und Y beide eine Bindung bedeuten oder m und n beide ungleich 1 sind, auch O, S, $SO_2$ oder $N-R_1$, oder, falls X und Y beide eine Bindung bedeuten, auch CO, gegebenenfalls in 2-Stellung durch eine Oxogruppe substituiertes 1,3-Cyclopentylen oder gegebenenfalls in 2-Stellung durch eine Oxogruppe und/oder in 5-Stellung durch Alkyl mit 1 bis 6 C-Atomen substituiertes 1,3-Cyclohexylen,

R und R' die gleich oder verschieden sein können, H, Halogen oder Alkyl mit 1 bis 6 C-Atomen und

$R_1$ H oder Alkyl mit 1 bis 6 C-Atomen

bedeuten,
mit der Maßgabe, daß $-X-C_mH_{2m}-Z-C_nH_{2n}-Y-$ nicht $-(CH_2)_2-$ oder $-(CH_2)_3-$ bedeutet, enthalten ist.

3. Verwendung von Bisazidoverbindungen nach Anspruch 1 als lichtempfindliche Komponente in negativ arbeitenden, für fernes UV-Licht empfindlichen Zusammensetzungen zum Erzeugen von Reliefstrukturen.

4. Verfahren zur fotolithographischen Herstellung von Reliefstrukturen durch Auftragen von lichtempfindlichen Zusammensetzungen in Form einer Schicht oder Folie auf ein Substrat, Bestrahlen der lichtempfindlichen Schicht durch eine Maske und Herauslösen oder Abziehen der nicht bestrahlten Schicht- oder Folienteile, dadurch gekennzeichnet, daß man eine negativ arbeitende, für fernes UV-Licht empfindliche Zusammensetzung nach Anspruch 2 verwendet und mit ferner UV-Strahlung belichtet.